# EUROPEAN PATENT SPECIFICATION

(11) **EP 3 970 714 B1**
(45) Date of publication and mention of the grant of the patent: **29.07.2026**
(21) Application number: 19929137.8
(22) Date of filing: 15.05.2019
(51) Int. Cl.: A61K 31/197, A61K 31/198, A61K 31/221, A61K 31/353, A61K 41/00, A61N 5/06, A61P 17/12, A61P 35/00

(54) **COMPOSITION COMPRISING A COMBINATION OF A PROTOPORPHYRIN IX (PPIX) PRECURSOR SELECTED FROM METHYL AMINOLEVULINATE (MAL) AND AMINOLEVULINIC ACID(ALA), ETHYLENEDIAMINETETRAACETIC ACID (EDTA) AND EPIGALLOCATECHIN GALATE (EGCG) FOR TOPICAL USE IN PHOTODYNAMIC THERAPY**
ZUSAMMENSETZUNG BESTEHEND AUS EINER KOMBINATION EINES PROTOPHORPHYRIN-IX-(PPIX)-VORLÄUFERS, AUSGEWÄHLT AUS METHYLAMINOLÄVULINAT (MAL) UND AMINOLÄVULINSÄURE (ALA), ETHYLENDIAMINTETRAESSIGSÄURE (EDTA) UND EPIGALLOCATECHINGALLAT (EGCG) ZUR TOPISCHEN ANWENDUNG IN DER PHOTODYNAMISCHEN THERAPIE.
COMPOSITION COMPOSÉE D'UNE COMBINAISON D'UN PRÉCURSEUR DE PROTOPHORPHYRINE IX (PPIX) CHOISI PARMI L'AMINOLÉVULINATE DE MÉTHYLE (MAL) ET L'ACIDE AMINOLÉVULINIQUE (ALA), D'ACIDE ÉTHYLÈNEDIAMINETÉTRAACÉTIQUE (EDTA) ET D'ÉPIGALLOCATÉCHINE GALLATE (EGCG) POUR UNE UTILISATION TOPIQUE DANS LE TRAITEMENT PAR THÉRAPIE PHOTODYNAMIQUE

(43) Date of publication of application: 23.03.2022
(73) Proprietor: Universidad De La Frontera, Temuco 4811230 (CL); León Garrido, Daniela Inés, Temuco, 4813299 (CL)
(72) Inventor: ILI GANGAS, Carmen Gloria, Villa Vista Volcán Temuco (CL); BREBI MIEVILLE, Priscilla Solange, 03191 Temuco (CL); ROA STRAUCH, Juan Carlos, Las Condes Santiago (CL); LEÓN GARRIDO, Daniela Inés, Temuco, 4813299 (CL)
(74) Representative: Clarke, Modet y Cía., S.L.
(86) International application number: PCT/IB2019/054042
(87) International publication number: WO 2020/229878

(56) References cited:
- WO-A2-2017/132178
- KR-B1- 101 133 770
- US-A1- 2014 128 797
- JORGE ANA ELISA S ET AL: "Photodynamic therapy improves the ultraviolet-irradiated hairless mice skin", PROGRESS IN BIOMEDICAL OPTICS AND IMAGING, SPIE - INTERNATIONAL SOCIETY FOR OPTICAL ENGINEERING, BELLINGHAM, WA, US, vol. 8926, 4 March 2014 (2014-03-04), pages 89260N - 89260N, XP060033158, ISSN: 1605-7422, ISBN: 978-1-5106-0027-0, DOI: 10.1117/12.2040297
- FORMAN A: "PHOTODYNAMIC THERAPY IN DERMATOLOGY: HISTORY AND HORIZONS", JOURNAL OF DRUGS IN DERMATOLOGY, STRATEGIC COMMUNICATION IN DERMATOLOGY, NEW YORK, NY, US, vol. 3, no. 1, SUPPL, 1 January 2003 (2003-01-01), pages S - 08, XP001191236, ISSN: 1545-9616
- CAPPUGI P ET AL: "TOPICAL 5-AMINOLEVULINIC ACID AND PHOTODYNAMIC THERAPY IN DERMATOLOGY: A MINIREVIEW", JOURNAL OF CHEMOTHERAPY, FLORENCE, IT, vol. 13, no. 5, 1 January 2001 (2001-01-01), pages 494 - 502, XP008030832
- XUE YANG ET AL: "Aminolevulinic Acid-Based Tumor Detection and Therapy: Molecular Mechanisms and Strategies for Enhancement", INTERNATIONAL JOURNAL OF MOLECULAR SCIENCES, vol. 16, no. 10, 28 October 2015 (2015-10-28), pages 25865 - 25880, XP055759593, DOI: 10.3390/ijms161025865
- ANGELA FERRARIO ET AL: "Pro-apoptotic and anti-inflammatory properties of the green tea constituent epigallocatechin gallate increase photodynamic therapy responsiveness : EGCG ENHANCES PDT RESPONSIVENESS", LASERS IN SURGERY AND MEDICINE., vol. 43, no. 7, 23 August 2011 (2011-08-23), US, pages 644 - 650, XP055759601, ISSN: 0196-8092, DOI: 10.1002/lsm.21081
- HANG QI ET AL: "(?)-Epigallocatechin-3-Gallate Ameliorates Photodynamic Therapy Responses in an In Vitro T Lymphocyte Model : EGCG IN PHOTODYNAMIC THERAPY", PHYSIOTHERAPY RESEARCH, vol. 28, no. 10, 3 April 2014 (2014-04-03), GB, pages 1486 - 1491, XP055759600, ISSN: 0951-418X, DOI: 10.1002/ptr.5152
- HANANIA J ET AL: "The effect of EDTA and serum on endogenous porphyrin accumulation and photodynamic sensitization of human K562 leukemic cells", CANCER LETTERS, NEW YORK, NY, US, vol. 65, no. 2, 14 August 1992 (1992-08-14), pages 127 - 131, XP026183554, ISSN: 0304-3835, [retrieved on 19920814], DOI: 10.1016/0304-3835(92)90156-P
- YANG, X. ET AL.: "Aminolevulinic Acid-Based Tumor Detection and Therapy: Molecular Mechanisms and Strategies for Enhancement", INT. J. MOL. SCI., vol. 16, 2015, pages 25865 - 25880, XP055759593, DOI: 1 0.3390/ijms1 61 025865
- QI, H. ET AL.: "Epigaliocatechin-3-Gallate Ameliorates Photodynamic Therapy Responses in an In Vitro T Lymphocyte Model", PHYTOTHERAPY RESEARCH, vol. 28, no. 10, 2014, pages 1486 - 1491, XP055759600, DOI: 10.1002/ptr.5152
- FERRARIO, A. ET AL.: "Pro-apoptotic and anti-inflammatory properties of the green tea constituent epigallocatechin gallate increase photodynamic therapy responsiveness", LASERS SURG MED., vol. 43, no. 7, September 2011 (2011-09-01), pages 644 - 650, XP055759601, DOI: 10.1002/lsm.21081.
- MUN, ST ET AL.: "Epigallocatechin gallate with photodynamic therapy enhances anti-tumor effects in vivo and in vitro", PHOTODIAGNOSIS AND PHOTODYNAMIC THERAPY, vol. 11, 2014, pages 141 - 147, XP011257083, DOI: 10.1016/j.pdpdt. 2014.03.00 3
- RAISH, M. ET AL.: "Photodynamic Therapy in Combination with Green Tea Polyphenol EGCG Enhances Antitumor Efficacy in Human Papillomavirus 16 (E6/E7) Immortalized Tumor Cells", THE JOURNAL OF APPLIED RESEARCH 2010, vol. 10, no. 2, pages 59 - 67, XP055760274

## Description

### FIELD OF THE INVENTION

The invention is directed to a photosensitising formulation that can be used in topical application for its use in photodynamic therapy (PDT), in the treatment of skin or mucous membranes.

### BACKGROUND OF THE INVENTION

Photodynamic therapy (PDT) is, generally speaking, a type of treatment for hyperproliferative diseases of the skin and internal epithelial, comprising the administration, by topical or systemic route, of a photosensible agent that will ideally be concentrated in the proliferating tissues of the body. The compound itself is inactive but after irradiation with light with a specific wavelength, the molecule is chemically activated and is stimulated for it to undergo chemical reactions that directly damage the cell or result in the production of species that are in turn harmful to the cells. In this way, the chemotherapeutic action is physically limited to an area of interest instead of extending to the whole body of the patient with unpleasant and harmful side effects. The application field of PDT is naturally limited by the accessibility from the tissue to the light source.

Among the hyperproliferative diseases of the skin, cancers can be found where only one of these, melanoma, is seriously life-threatening, and is not a candidate for PDT. Among the non-melanoma skin cancers (NMSC), basal cell carcinoma is found (80% of the cases of NMSC), relatively benign, and squamous cell carcinoma (20% of the cases of NMSC) that have an intermediate danger since they can occasionally metastasize (see World Health Organization. Ultraviolet radiation and the INTERSUN Programme. http://www.who.int/uv/faq/skincancer/en/index1.html (2013)). Hyperplasias, such as actinic keratosis and Bowen's disease, are referred to as precancerous lesions given that these can cause squamous cell carcinoma if not treated.

For this type of lesions or diseases, photodynamic therapy is useful. In practical terms, in order to carry out this dermatological therapy is applied, topically, a formulation comprising a photosensitising (PS) compound, where this compound when irradiated or excited to a light with a specific wavelength generates reactive oxygen species (ROS), which are cytotoxic and induce the death of the cells in which they are found. In dermatology, the common procedure is applying a PS on the skin lesion to be treated, for example, by means of a cream, then letting it incubate and, finally, radiating the area with light for it to generate on-site ROS and the hyperproliferative tissue to be removed.

The invention corresponds to a composition for using in photodynamic therapy (PDT) on the skin, for one of its uses is in the clinical field, specifically, dermatological. This therapy consists of applying the photosensitising cream on the skin lesions and then radiating said lesions with a light of specific wavelength. PDT allows for the treatment of lesions from non-melanoma skin cancer, specifically, basal cell carcinoma and precursor lesions of squamous cell carcinoma of the skin. These lesions have a high incidence worldwide, since these are produced in skin areas exposed to the sun such as, for example, the face. It is estimated that, around the world, there are 2-3 million new cases of this type of cancer each year (see World Health Organization. Ultraviolet radiation and the INTERSUN Programme. http://www.who.int/uv/faq/skincancer/en/index1.html (2013)).

However, some tissues prove to be resistant to this type of therapies (PDT), or the treatment does not adequately reach the whole diseased tissue. For this reason, the development of new compositions that enhance the effect from PSs becomes necessary, this in order to improve the results of the PDT.

In addition, there are other applications for this type of therapy, where the photodynamic therapy is used in the treatment and prevention of inflammatory and infectious diseases of the skin and mucous membranes, and in applications with purely dermocosmetic purposes as well.

WO2017/132178 discloses salts of 5-aminolevulinic acid and esters thereof in photodynamic therapy. The document discloses lists of possible ingredients of the composition, including EDTA to increase the accumulation and absorption of PpIX, chelating ferrous ions. The document does not disclose any example or composition comprising a specific concentration of EDTA.

US 2014/128797 describes the production of a PDT cream comprising ALA and amino acids as a photosensitizer for acne treatment and skin improvement. It uses green tea extract as one of its excipients, but not as an active compound. This document is silent regarding EDTA or EGCG.

KR 101133770 discloses PDT using ALA and green tea extract for the treatment of skin aging due to UV light exposure. The compositions of D3 does not disclose EGCG.

Jorge AES et al., 2014, Progress in Biomedical Optics and Imaging, International Society for Optical Engineering, vol. 8926, p. 89260N-89260N discloses photodynamic therapy (PDT) as emerging modality for cancer and other skin conditions, using ALA. This document discloses EDTA 1% as chelating agent, but it is silent regarding the presence of EGCG.

Forman A, 2003, Journal of Drugs in Dermatology, Strategic Communication in Dermatology, vol. 3, no. 1, p. S-08 discloses the use of ALA in PDT for the treatment of dermatology diseases. This document is a complete review of possible treatments for PDT-associated conditions but is silent regarding EDTA or EGCG.

Cappugi P et al., 2001, Journal of Chemotherapy, vol. 13, no. 5, 2001, pages 494-502 discloses application of ALA in PDT related with cancer and suggest the use of EDTA for Bowen's disease treatment. For other diseases such as Basal cell carcinoma, EDTA has to be applied before PDT. This document is silent regarding any specific concentration of EDTA in combination with the photosensitizer or the presence of EGCG.

Yang X et al., 2015, International Journal of Molecular Sciences, vol. 16, no. 10, 2015, p. 25865-25880 discloses ALA in PDT. The document is focused in PpIX production, mechanisms involved enhancing PplX production, etc. The author describes iron chelators for the accumulation of PpIX, such as EDTA. It is remarkable that this document is silent on EGCG, which confirms that the combination of EDTA and EGCG is not obvious.

Ferrario A et al., 2011, Lasers in Surgery and Medicine, vol. 43, no. 7, 2011, p. 644-650 discloses the pro-apoptotic and anti-inflammatory properties of EGCG, a constituent of green tea. The authors suggest that this element might be used in PDT. 4 mg/mL EGCG is combined with 25 µg/mL or 0.5 mg/mL pf photofrin profiler sodium. D8 is silent on EDTA and uses high concentrations.

Qi H et al., 2014, Physiotherapy Research, vol. 28, no. 10, 2014, p. 1486-1491 discloses EGCG as having both antioxidant and prooxidant properties, and its effect in PDT. The document assays the effect of the compound alone.

Hanania J et al., 1992, Cancer Letters, vol. 65, no. 2, 1992, pages 127-131, discloses the effect of EDTA and serum on endogenous porphyrin accumulation and photodynamic sensitization in tumor cells treated with 5-ALA.

In this way, the present invention is related to the pharmaceutical and dermocosmetic industries. Particularly, it is related to a composition for photodynamic therapy that increases its scope, enhancing the effect from known photosensitising compounds, such as the compound methyl aminolevulinate (MAL) or aminolevulinic acid (ALA), for example.

### STATE OF THE ART

In the state of the art are different photosensitisers for photodynamic therapies, where a group of these are protoporphyrin IX (PpIX) precursors, such as methyl aminolevulinate (MAL) or aminolevulinic acid (ALA), which are broadly used for dermatological lesions, such as those that the present invention is directed to.

On 2015, Yang *et al.* (Yang, X., Palasuberniam, P., Kraus, D., & Chen, B. (2015) International Journal of Molecular Sciences, 16(10), 25865-25880) published that, ferric ion scavengers could enhance the use if this photosensitiser, since said ion is a substrate necessary for the PpIX conversion pathway in hemoglobin, which led to the incorporation of ferric ion chelating agents in photosensitising compositions, in order to improve the therapy. An agent used for this purpose is EDTA.

On the other hand, Mun (Mun ST, Bae DH, Ahn WS. Photodiagnosis Photodyn Ther. 2014. Jun;11(2):141-7.) suggests that PDT combined with EGCG could be useful for an effective cancer treatment. Due to the fact that it was observed both a decrease in the TC-1 cell line growth (derived from hybridoma) and from the tumors from this line generated in C57BL/6 mice, after having treated them with PDT + EGCG using Radachlorin as photosensitiser. In this work, EGCG is not applied directly with the photosensitiser in the *in vivo* assays as is carried out in the invention, but rather was administered by injection in the tumors by 20 days after PDT, with which they observed the best results regarding the decrease in the size of the tumors in mice. Therefore, the results obtained are based on a treatment where EGCG is applied after PDT, not simultaneously with the photosensitiser.

Although these therapies already have several years of development, complete response rates to the treatment have been reported which vary from a 37% to 89%, according to the type of lesion treated and follow-up time to therapy. Consequently, there is a significant percentage of recurrence or resistance to the treatment by PDT in this type of lesions, even with this knowledge available.

### SOLUTION TO THE TECHNICAL PROBLEM

To remedy the problem raised, the inventors have developed a new pharmaceutical and dermocosmetic composition for topical use prepared based on an protoporphyrin IX (PpIX) photosensitiser, in particular, methyl aminolevulinate (MAL) or aminolevulinic acid (ALA), combined with ethylenediaminetetraacetic acid (EDTA) and epigallocatechin galate (EGCG). These new formulations show an enhancing effect of the effect of the photosensitising compounds, MAL or ALA, for example, which ensures a greater efficiency of photodynamic therapy, for example, in the resolution of long-term dermatological lesions or of preneoplastic or neoplastic mucous membranes, for example, preneoplastic lesions of the cervix (intraepithelial lesions of the cervix) decreasing the recurrence rate of these lesions. Or in any other application of photodynamic therapy, such as the treatment or prevention of inflammatory diseases, on the skin or mucous membranes, for example, acne, rosacea, cellular rejuvenation, or infectious diseases on the skin or mucous membranes.

### DESCRIPTION OF THE DRAWINGS

**Figure 1****. Effect of EDTA and EGCG on MAL - PDT on the viability of PDT-resistant HSC-1 cells assessed by MTT.** A) Effect of EGCG associated to PDT. B) Effect of EDTA associated to PDT. C) and D) Controls without light of EGCG and EDTA, respectively. The results represent the ± DS average. The experiments were carried out in technical and biological triplicate. *P < 0.05.
**Figure 2****. Effect of the formulation from the invention, that combines EDTA and EGCG on MAL-PDT on the viability of PDT-resistant HSC-1 cells assessed by MTT.** The results represent the ± DS average. The experiments were carried out in technical and biological triplicate. *P < 0.05.
**Figure 3****. Detection of protoporphyrin IX (PpIX) in PDT-resistant HSC-1 cells by flow cytometry.** A) Effect of EGCG in the PplX synthesis. B) Effect of EDTA in the PpIX synthesis. The experiments were carried out in technical and biological triplicate. **P < 0.005.
**Figure 4****. Detection of reactive oxygen species (ROS) in PDT-resistant HSC-1 cells by flow cytometry.** A) Effect of EGCG in the generation of ROS. The experiments were carried out in technical and biological triplicate. *P < 0.005.

### DESCRIPTION OF THE INVENTION

Photodynamic therapy, as already mentioned, consists of the application of a cream on the skin lesion and then radiated with a specific light. The cream contains a photosensitiser that enters the cell and is accumulated in it. When the lesion is exposed to the specific light, the photosensitiser reacts with the O₂ present in the cell and forms reactive oxygen species that cause cell damage, and consequently cell death. It is an outpatient treatment, which can be applied over large areas of the skin but, above all, its greatest advantage is that optimum cosmetic results are obtained, which is important, considering that many times lesions are developed in areas exposed to the sun. This type of therapies has multiple applications, for example, in lesions of the skin or non-melanoma neoplastic or preneoplastic mucous membranes, in addition this therapy is also employed in the treatment and prevention of inflammatory diseases, on the skin or mucous membranes, for example, acne, rosacea, cellular rejuvenation, or infectious diseases on the skin or mucous membranes.

However, this therapy is not always effective, for example, in non-melanoma neoplastic or preneoplastic lesions, there is a significant rate of recurrences or resistance to the treatment by PDT in this type of lesions.

The invention is directed to new photosensitising pharmaceutical and dermocosmetic formulations of topical application for its use in photodynamic therapy (PDT), based on PpIX precursors, photosensitisers selected from methyl aminolevulinate (MAL) and/or aminolevulinic acid (ALA), combined with ethylenediaminetetraacetic acid (EDTA) and epigallocatechin galate (EGCG). This new combination will ensure a greater efficiency of photodynamic therapy, especially in the treatment of non-melanoma skin cancer, in the resolution of preneoplastic or long-term neoplastic dermatological lesions, decreasing the recurrence rate of these lesions.

The inventors have included in the composition of the invention, EDTA, given that this compound has a chelating effect that contributes to the increase of the cytotoxic effect allowing the PplX cellular accumulation (compound synthesized by the cells from MAL and ALA), thus increasing the production of reactive oxygen species (ROS) that cause cell damage.

On the other hand, the inventors have included EGCG, given that its presence in the composition boosts and strengthens the efficiency of PDT, has a chelating, prooxidant, and antiproliferative effect. In this way, the composition of the invention allows for PDT to improve its efficiency ensuring the destruction of preneoplastic and neoplastic cells of non-melanoma skin cancer.

Surprisingly, these new combinations have a highly superior effect to that produced by the photosensitiser (ALA or MAL) without the enhancers, which allows for the improvement of the efficiency of the therapy and decrease of recurrence of already treated lesions.

The composition contains the photosensitiser (MAL or ALA) at a concentration between 100 mg/g - 200 mg/g, EDTA at a concentration between 0.5 mg/g - 10 mg/g and EGCG at a concentration between 0.1 - 10 mg/g, in pharmacologically acceptable carriers and/or excipients.

Where photosensitisers, MAL or ALA can be found freely or from a pharmacologically acceptable salt, such as for example, methyl aminolevunilate hydrochloride, aminolevunilic acid methyl ester or methyl 5-amino-4-oxopentanoate hydrochloride, among others.

The excipients are selected from water, sodium chloride, lanolin, beeswax, glycerol, petroleum jelly, propylene glycol, sodium lauryl sulfate, dimethyl sulfoxide, imidazolidinyl urea, olivem 1000, propyl parahydroxybenzoate, Polawax, methylparaben, almond oil, castor oil, cetyl alcohol, butylhydroxytoluene, and any pther excipient available in the art.

In addition, the composition of the invention may contain other active compounds or formulation aids.

The composition of the invention may be employed in any application of photodynamic therapy that there is in the art, for example, in pharmaceutical or dermocosmetic applications.

In an embodiment, the composition of the invention may be employed to prepare a drug product useful for photodynamic therapy, useful in the treatment of preneoplastic and neoplastic cells of non-melanoma skin cancer. In a preferred embodiment, the composition of the invention is employed in the treatment of photodynamic therapy-resistant cells or lesions.

In addition, the composition of the invention may be employed in the treatment or prevention of inflammatory and/or infectious diseases on the skin or mucous membranes, for example, acne or rosacea, among others

In another embodiment, the composition of the invention may be employed in photodynamic therapy for dermocosmetic purposes, for example, for cellular rejuvenation.

The composition of the invention may be constituted in different pharmaceutical or dermocosmetic presentations, such as cream, ointment, spray, lotion, foam, or any other existing in the art.

The scope of the invention will become clearer in light of the examples given, which should be considered as illustrative, and under no circumstance limiting of the present invention.

### APPLICATION EXAMPLES

### EXAMPLE 1.

### In vitro effect of EGCG and EDTA separately as adjuvants of MAL-PDT

The *in vitro* effect of EGCG and EDTA separately as adjuvants of MAL-PDT was assessed HSC-1 cells derived from squamous skin carcinoma were used. Previously, these cells received PDT cycles, in order to select those that are resistant to PDT. Therefore, the model used corresponds to MAL - PDT-resistant HSC-1 cells.

The *in vitro* photodynamic conventional treatment consisted of incubating these cells with MAL 150 mg/g (photosensitiser) for 4 hours in the dark and after radiating them with 630 nm red light, with fluence of 4 J/cm². Cell viability was assessed 24 hours later by the MTT assay.

Under these MAL-PDT conditions, which are the control conditions, as a result it was obtained that approximately a 50% of HSC-1 resistant cells survived PDT. Subsequently, this protocol was carried out in the same way in these cells, only with the following modification: when adding MAL, different concentrations of EDTA were added (0.5 mg/g, 1 mg/g, 1.5 mg/g, and 2 mg/g) or EGCG (0.2 mg/g, 0.4 mg/g, 0.6 mg/g, 0.8 mg/g). The MAL concentration of 150 mg/g was kept in all tests and experiments with irradiation (light) fluency same to the control at 4 J/cm², or without radiation, were carried out.

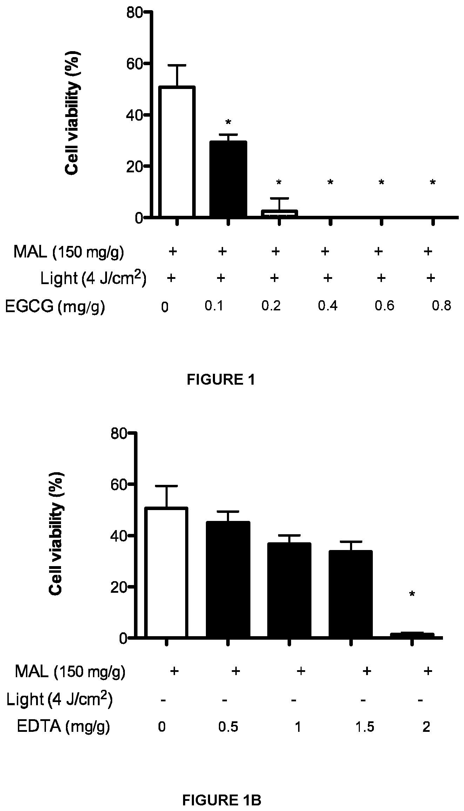

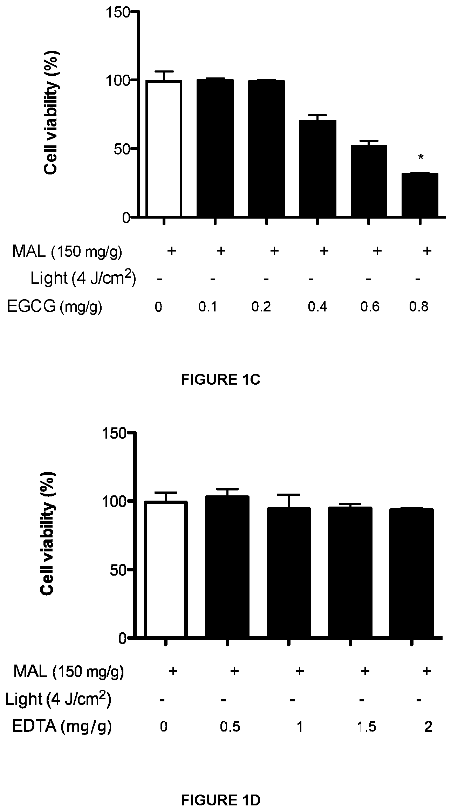

The effect of EGCG on MAL - PDT is observed in Figure 1A (light) and 1C (controls without light). In all tested EGCG concentrations, under light conditions, the viability of resistant cells decreased significantly, even from 0.4 mg/g, the survival rate was 0. In turn, the effect of EDTA only at a 2 mg/g concentration in MAL-PDT was significant, the results are plotted in Figure 1B (light) and 1D (controls without light).

### EXAMPLE 2

### In vitro effect of EGCG and EDTA combined as adjuvants of MAL-PDT

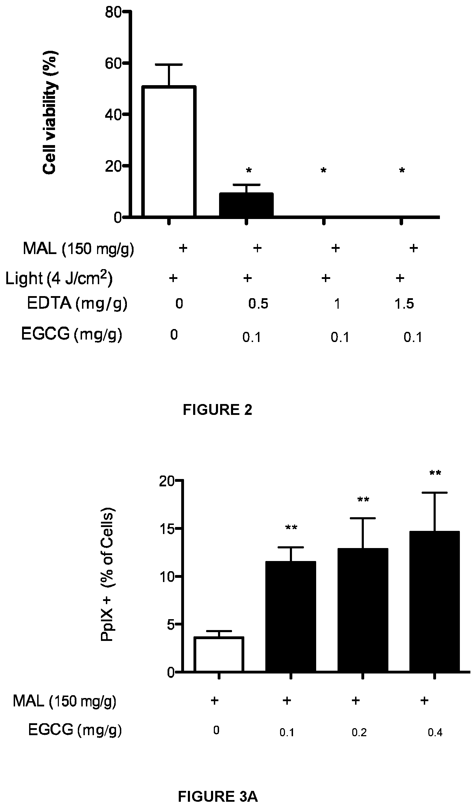

The synergetic effect of the combination of the invention EGCG + EDTA in MAL-PDT was assessed similarly to that discussed in example 1 but with lower EGCG concentrations. In Figure 2, the effect of 0.5 mg/g, 1 mg/g, and 1.5 mg/g EDTA, in presence of 0.1 mg/g EGCG, as MAL-PDT enhancers in HSC-1 resistant cells, was shown.

The results show that all compositions of the invention had a very significant decrease regarding the control. In the lowest concentrations assessed (10 mg/g EGCG, 0. 5 mg/g EDTA, 150 mg/g MAL), cell viability is of only 10%, and under the other 2 conditions there is a 0% viability of MAL-PDT-resistant cells.

### EXAMPLE 3

### Detection of protoporphyrin IX (PpIX) in PDT-resistant HSC-1 cells when incubated with MAL and EGCG or EDTA

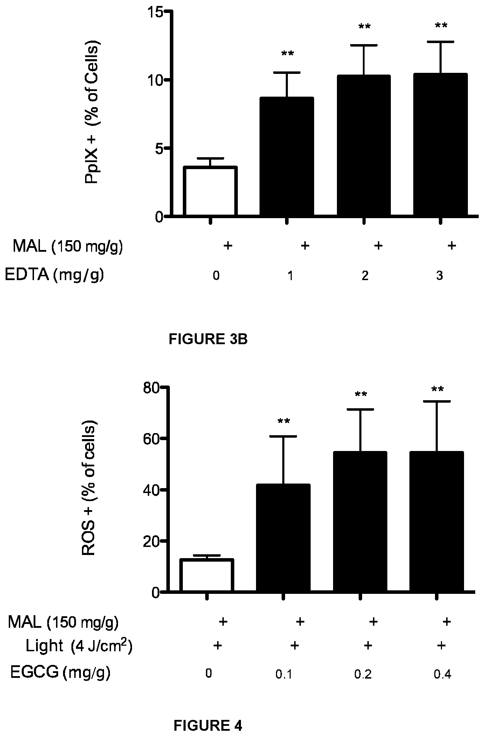

In order to understand the mechanisms of action of the compositions of the invention and, due to the fact that EGCG and EDTA have chelating capability, it was assessed if the presence of these compounds would increase the content of PpIX in cells. For this, PDT-resistant cells, obtained as stated in example 1, were incubated with 150 mg/g MAL, plus EGCG (0.1 mg/g, 0.2 mg/g, 0.4 mg/g) or EDTA (1 mg/g, 2 mg/g, 3 mg/g) for 4 hours in the dark. Subsequently, the PplX content in resistant cells was detected by flow cytometry, since PplX is a fluorescent compound. The results show that both compounds significantly increase the production of PpIX. As it can be clearly observed in Figure 3, when incubating PDT-resistant cells with MAL, only a 5% of the population contained PpIX, while in presence of EDTA (Figure 3B) or EGCG (Figure 3A) this percentage increases up to a 13 or 18%, respectively.

### EXAMPLE 4

### Detection of reactive oxygen species (ROS) in PDT-resistant HSC-1 cells when incubated with MAL and EGCG

Given that in PDT, it is finally the reactive oxygen substances that cause cell damage and death, the production of these compounds was directly assessed. The same model as in example 3 was used, at the same EGCG concentrations stated in Figure 3A, in order to assess the production of ROS generated in MAL-PDT. Following the previous methodology, after incubating the cells for 4 hours, these received red light irradiation. For ROS detection, these were incubated with a probe (Muse^{™} Oxidative Stress Kit | MCH100111 - Merck Millipore) for 30 minutes and was assessed in a cell analyzer. The obtained results show that the presence of EGCG in MAL-PDT stimulates the productions of RROS in cells, given that this compound gas a prooxidant effect (Figure 4). In all assessed EGCG concentrations, the ROS concentration doubled or even tripled the concentration obtained in the control.

Therefore, based on the results described in the examples above, it is shown that the compositions of the invention containing EGCG and EDTA enhance the cytotoxic effect of MAL-PDT in HSC-1 cells resistant to the treatment. On the other hand, the synergetic effect of both compounds may be observed even when using very low concentrations of EGCG, due to the fact that the effect of EGCG is greater than that of EDTA.

**Statistical Analysis:** the data was presented as the ± DS average and the significance was tested with the Mann Whitney test using GraphPad Prism (GraphPad Software, La Jolla, CA, USA). All assays were carried out using technical and biological triplicate. The statistical significance was established with a P value < 0.05.

## Claims

1. Composition for photodynamic therapy, wherein it comprises a photosensitiser which is a protoporphyrin IX (PpIX) precursor at a concentration between 100 mg/g - 200 mg/g combined with ethylenediaminetetraacetic acid (EDTA) at a concentration between 0.5 mg/g - 10 mg/g and epigallocatechin galate (EGCG) at a concentration between 0.1 mg/g - 10 mg/g, in pharmacologically acceptable carriers and/or excipients, and wherein the protpporphyrin IX (PpIX) precursor is selected from methyl aminolevulinate (MAL) and/or aminolevulinic acid(ALA) and its pharmacologically acceptable salts.

2. Composition according to claim 1, wherein the pharmacologically acceptable carriers and/or excipients are for a topical application and are selected from water, sodium chloride, lanolin, beeswax, glycerol, petroleum jelly, propylene glycol, sodium lauryl sulfate, dimethyl sulfoxide, imidazolidinyl urea, olivem 1000, propyl parahydroxybenzoate, Polawax, methylparaben, almond oil, castor oil, cetyl alcohol, butylhydroxytoluene.

3. Composition according to claim 2, wherein it may be constituted of different pharmaceutical or dermocosmetic presentations such as cream, ointment, spray, lotion, foam.

4. Composition according to any of claims 1 to 3 for use in photodynamic therapy.

5. The composition for use according to claim 4, wherein the photodynamic therapy comprises the treatment of preneoplastic and neoplastic cells of non-melanoma skin cancer.

6. The composition for use according to claim 5, wherein the photodynamic therapy comprises the treatment of cells or lesions resistant to photodynamic therapy.

7. The composition for use according to claim 4, wherein the photodynamic therapy comprises the treatment or prevention of inflammatory diseases on the skin or mucous membranes.

8. The composition for use according to claim 7, wherein the inflammatory diseases include acne, rosacea.

9. The composition for use according to claim 4, wherein the photodynamic therapy comprises the treatment or prevention of infectious diseases on the skin or mucous membranes.

10. Non-therapeutic use of the composition according to any of claims 1 to 3, wherein it serves for preparing compositions for photodynamic therapy for dermocosmetic purposes.

11. The non-therapeutic use according to claim 10, wherein the dermocosmetic composition is useful for cellular rejuvenation.

## Patentansprüche

1. Zusammensetzung für die photodynamische Therapie, wobei sie einen Photosensibilisator umfasst, der ein Protoporphyrin-IX(PpIX)-Vorläufer ist, in einer Konzentration zwischen 100 mg/g-200 mg/g, kombiniert mit Ethylendiamintetraessigsäure (EDTA) in einer Konzentration zwischen 0,5 mg/g-10 mg/g sowie Epigallocatechingallat (EGCG) in einer Konzentration zwischen 0,1 mg/g-10 mg/g, in pharmakologisch verträglichen Trägern und/oder Hilfsstoffen, und wobei der Protoporphyrin-IX (PpIX)-Vorläufer aus Methylaminolevulinat (MAL) und/oder Aminolevulinsäure (ALA) und deren pharmakologisch verträglichen Salzen ausgewählt ist.

2. Zusammensetzung nach Anspruch 1, wobei die pharmakologisch verträglichen Träger und/oder Hilfsstoffe für eine topische Anwendung bestimmt sind und aus Wasser, Natriumchlorid, Lanolin, Bienenwachs, Glycerin, Vaseline, Propylenglykol, Natriumlaurylsulfat, Dimethylsulfoxid, Imidazolidinylharnstoff, Olivem 1000, Propylparahydroxybenzoat, Polawax, Methylparaben, Mandelöl, Rizinusöl, Cetylalkohol und Butylhydroxytoluol ausgewählt sind.

3. Zusammensetzung nach Anspruch 2, wobei sie aus verschiedenen pharmazeutischen oder dermokosmetischen Darreichungsformen wie Creme, Salbe, Spray, Lotion oder Schaum bestehen kann.

4. Zusammensetzung nach einem der Ansprüche 1 bis 3 zur Verwendung in der photodynamischen Therapie.

5. Zusammensetzung zur Verwendung nach Anspruch 4, wobei die photodynamische Therapie die Behandlung von präneoplastischen und neoplastischen Zellen bei nicht-melanotischem Hautkrebs umfasst.

6. Zusammensetzung zur Verwendung nach Anspruch 5, wobei die photodynamische Therapie die Behandlung von Zellen oder Läsionen umfasst, die gegenüber der photodynamischen Therapie resistent sind.

7. Zusammensetzung zur Verwendung nach Anspruch 4, wobei die photodynamische Therapie die Behandlung oder Vorbeugung von entzündlichen Erkrankungen der Haut oder der Schleimhäute umfasst.

8. Zusammensetzung zur Verwendung nach Anspruch 7, wobei die entzündlichen Erkrankungen Akne und Rosazea umfassen.

9. Zusammensetzung zur Verwendung nach Anspruch 4, wobei die photodynamische Therapie die Behandlung oder Vorbeugung von Infektionskrankheiten der Haut oder der Schleimhäute umfasst.

10. Nichttherapeutische Verwendung der Zusammensetzung nach einem der Ansprüche 1 bis 3, wobei sie zur Herstellung von Zusammensetzungen für die photodynamische Therapie zu dermokosmetischen Zwecken dient.

11. Nichttherapeutische Verwendung nach Anspruch 10, wobei die dermokosmetische Zusammensetzung zur Zellverjüngung geeignet ist.

## Revendications

1. Composition pour thérapie photodynamique, dans laquelle elle comprend un photosensibilisateur qui est un précurseur de la protoporphyrine IX (PpIX) à une concentration comprise entre 100 mg/g et 200 mg/g combiné avec de l'acide éthylènediaminetétraacétique (EDTA) à une concentration comprise entre 0,5 mg/g et 10 mg/g et du gallate d'épigallocatéchine (EGCG) à une concentration comprise entre 0,1 mg/g et 10 mg/g, dans des véhicules et/ou excipients pharmacologiquement acceptables, et dans laquelle le précurseur de la protoporphyrine IX (PpIX) est choisi parmi l'aminolévulinate de méthyle (MAL) et/ou l'acide aminolévulinique (ALA) et ses sels pharmacologiquement acceptables.

2. Composition selon la revendication 1, dans laquelle les véhicules et/ou excipients pharmacologiquement acceptables sont destinés à une application topique et sont choisis parmi l'eau, le chlorure de sodium, la lanoline, la cire d'abeille, le glycérol, la vaseline, le propylène glycol, le laurylsulfate de sodium, le diméthylsulfoxyde, l'urée imidazolidinyle, Olivem 1000, le parahydroxybenzoate de propyle, Polawax, le méthylparabène, l'huile d'amande, l'huile de ricin, l'alcool cétylique, le butylhydroxytoluène.

3. Composition selon la revendication 2, dans laquelle elle peut être constituée de différentes présentations pharmaceutiques ou dermocosmétiques telles qu'une crème, une pommade, un spray, une lotion, une mousse.

4. Composition selon l'une quelconque des revendications 1 à 3 pour une utilisation en thérapie photodynamique.

5. Composition pour une utilisation selon la revendication 4, dans laquelle la thérapie photodynamique comprend le traitement de cellules prénéoplasiques et néoplasiques de cancer cutané non-mélanome.

6. Composition pour une utilisation selon la revendication 5, dans laquelle la thérapie photodynamique comprend le traitement de cellules ou de lésions résistantes à la thérapie photodynamique.

7. Composition pour une utilisation selon la revendication 4, dans laquelle la thérapie photodynamique comprend le traitement ou la prévention de maladies inflammatoires de la peau ou des muqueuses.

8. Composition pour une utilisation selon la revendication 7, dans laquelle les maladies inflammatoires comprennent l'acné, la rosacée.

9. Composition pour une utilisation selon la revendication 4, dans laquelle la thérapie photodynamique comprend le traitement ou la prévention de maladies infectieuses de la peau ou des muqueuses.

10. Utilisation non thérapeutique de la composition selon l'une quelconque des revendications 1 à 3, dans laquelle elle sert à préparer des compositions pour thérapie photodynamique à des fins dermocosmétiques.

11. Utilisation non thérapeutique selon la revendication 10, dans laquelle la composition dermocosmétique est utile pour le rajeunissement cellulaire.
